(19) Europäisches Patentamt
European Patent Office
Office européen des brevets

(11) EP 2 262 820 B1

(12) # EUROPÄISCHE PATENTSCHRIFT

(45) Veröffentlichungstag und Bekanntmachung des Hinweises auf die Patenterteilung:
**19.06.2013 Patentblatt 2013/25**

(21) Anmeldenummer: **09727714.9**

(22) Anmeldetag: **03.04.2009**

(51) Int Cl.:
***C07F 9/6564*** (2006.01)

(86) Internationale Anmeldenummer:
**PCT/DE2009/000437**

(87) Internationale Veröffentlichungsnummer:
**WO 2009/121347 (08.10.2009 Gazette 2009/41)**

(54) **VERFAHREN ZUR STEREOSELEKTIVEN SYNTHESE VON PHOSPHORVERBINDUNGEN**

METHOD FOR THE STEREOSELECTIVE SYNTHESIS OF PHOSPHORUS COMPOUNDS

PROCÉDÉ DE SYNTHÈSE STÉRÉOSÉLECTIVE DE COMPOSÉS PHOSPHORÉS

(84) Benannte Vertragsstaaten:
**AT BE BG CH CY CZ DE DK EE ES FI FR GB GR HR HU IE IS IT LI LT LU LV MC MK MT NL NO PL PT RO SE SI SK TR**

(30) Priorität: **04.04.2008 DE 102008017500**

(43) Veröffentlichungstag der Anmeldung:
**22.12.2010 Patentblatt 2010/51**

(73) Patentinhaber: **Universität Hamburg**
**20146 Hamburg (DE)**

(72) Erfinder:
- **MEIER, Chris**
**21635 Jork (DE)**
- **THOMANN, Jens**
**49692 Cappeln (DE)**

(74) Vertreter: **Stüven, Ralf**
**Pohl & Partner**
**Patentanwälte**
**Kirchenhang 32 b**
**21073 Hamburg (DE)**

(56) Entgegenhaltungen:
**WO-A-2008/032531 JP-A- 2002 080 429**

Bemerkungen:
Die Akte enthält technische Angaben, die nach dem Eingang der Anmeldung eingereicht wurden und die nicht in dieser Patentschrift enthalten sind.

EP 2 262 820 B1

Anmerkung: Innerhalb von neun Monaten nach Bekanntmachung des Hinweises auf die Erteilung des europäischen Patents im Europäischen Patentblatt kann jedermann nach Maßgabe der Ausführungsordnung beim Europäischen Patentamt gegen dieses Patent Einspruch einlegen. Der Einspruch gilt erst als eingelegt, wenn die Einspruchsgebühr entrichtet worden ist. (Art. 99(1) Europäisches Patentübereinkommen).

Printed by Jouve, 75001 PARIS (FR)

## Beschreibung

Die Erfindung betrifft ein Verfahren zur stereoselektiven Synthese von Phosphorverbindungen, insbesondere von Phosphattriestern.

Chirale Phosphorverbindungen sind z.B. bei der Katalyse als Liganden, im Pflanzenschutz oder auch in der Medizin von großer Bedeutung. CycloSaligenylNucleosidmonophophate (cycloSal-NMPs) beispielsweise bilden eine Gruppe von Nucleotid-Prodrugs, die zur Bekämpfung viraler Erkrankungen (AIDS, Herpes, Epstein-Barr-Virus, Hepatitis, Varizella Zoster Virus) herangezogen werden können (c. Meier; MiniRev. Med. Chem. 2002, 2, 219-234). Mit den bisher im Stand der Technik bekannten Synthesestrategien fallen alle cycloSal-NMPs jedoch als Diastereomerengemische an (C. Meier; Eur. J. Drg. Chem., 2006, 1081-1102). Nur in den wenigsten Fällen ist eine Trennung in reine Stereoisomere möglich. In diesen Fällen konnte gezeigt werden, dass die getrennten Diastereomere teils erhebliche Unterschiede in ihren Eigenschaften in Bezug auf z.B. antivirale Aktivität, Hydrolysestabilität und Toxizität aufweisen (C. Meier, M. Lorey, E. De Clercq, J. Balzarini; J. Med. Chem., 1998, 41, 14171427).

Die Zulassung eines Stereoisomerengemisches als pharmazeutischer Wirkstoff ist im Vergleich zur Zulassung einer stereoisomerenreinen Verbindung durch die erhebliche Ausweitung der durchzuführenden physiologischen Untersuchungen (Adsorption, Distribution, Metabolismus und Exkretion jedes einzelnen Stereoisomers und der Isomerengemische) erheblich aufwändiger und kostenintensiver. Das führte in den letzen Jahren zu einer Zunahme der Zulassung stereoisomerenreiner Wirkstoffe im Vergleich zu Stereoisomerengemischen.

Zur Synthese chiraler Phosphorverbindungen sind im Stand der Technik grundsätzlich drei Strategien bekannt:

1. Asymmetrische Synthese
2. Trennung der Stereoisomere
3. Enzymatische Transformation

Bei der asymmetrischen Synthese verläuft die gängigste Route zur Darstellung von isomerenreinen P(III)- und P(IV)-Verbindungen über die Einführung eines bidenten chiralen Auxiliars, das Schritt für Schritt durch die gewünschten Substituenten vom Phosphoratom verdrängt wird (s. C.R. Hall, T.D. Inc; Phosphorus, Sulfur & related Elements, 1979, 7, 171-84). Kommerziell verfügbare oder leicht zugängliche Aminoalkohole und Zuckerderivate dienen als Leitstruktur dieser chiralen Auxiliare. L-Ephedrin wird beispielsweise häufig als ein solches chirales Auxiliar eingesetzt. Da dessen Abgabe in Deutschland jedoch dem Grundstoffüberwachungsgesetz unterliegt, ist die Verwendung aufgrund des erhöhten bürokratischen Aufwandes meist auf akademische Aufgabenstellungen beschränkt. Darüber hinaus stellt der P-O-Bindungsbruch eine starke Einschränkung dar, so dass heute nur die asymmetrische Synthese diastereomerenreiner Thioate möglich ist. Diese Synthesestrategie versagt aber bei stereoisomerenreinen Phosphattriestern.

Bei der Stereoisomerentrennung werden zur Darstellung separierbarer Diastereomerengemische sehr oft Derivate der natürlich vorkommenden Aminosäure L-Prolin und dessen Antipode D-Prolin verwendet (S. Ryu, J.A. Jackson, C.M. Thompson; J. Org. Chem. 1991, 56, 4999-5002). Generell werden reaktive Phosphorverbindungen mit diesen Prolinderivaten zu einem Phosphorsäureamidat umgesetzt, das durch Kristallisation oder chromatographische Trennung in seine Diastereomere getrennt werden kann. Der Nachteil dieser Strategie liegt einerseits in der atomökonomischen Ineffizienz (max. 50% Ausbeute, ein Diastereomer muss stets verworfen werden), andererseits sind nur strukturell einfache Phosphattriester darstellbar. So ist die Verwendung säurelabiler Substituenten nicht möglich.

Der Einsatz von Enzymen zur kinetischen Racematspaltung von chiralen Phosphorsäureanaloga ist ein relativ junges Forschungsgebiet, so dass bisher nur wenige Ansätze veröffentlicht wurden (s. z.B. F. Wu, W.-S. Li, M. Chen-Goodspeed, M.A. Sogorb, F.M. Raushel; J. Am. Chem. Soc. 2000, 122, 10206-07). Auch hier liegt ein Nachteil in der ineffizienten Atomökonomie, da das ungewünschte Isomer zur Hälfte entsteht und durch entsprechende Enzyme vom gewünschten Isomer getrennt werden muss. Ferner müssen die Enzyme durch Mutation auf die jeweiligen Moleküle optimiert werden.

Es besteht somit ein hoher Bedarf an einem verbesserten Verfahren zur Darstellung isomerenreiner chiraler Phosphorverbindungen, beispielsweise chiraler Organophosphorverbindungen wie Phosphattriestern. Damit liegt der vorliegenden Erfindung die Aufgabe zugrunde, ein solches Verfahren bereitzustellen, das die Nachteile des Standes der Technik nicht aufweist und insbesondere atomökonomisch effizienter ist als die bisherigen Verfahren.

Erfindungsgemäß wird diese Aufgabe durch das Verfahren mit den Merkmalen des Anspruchs 1 gelöst.

Die Erfindung stellt ein Verfahren zur stereoselektiven Synthese von Phosphorverbindungen der Formel I oder II

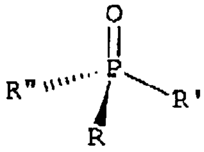 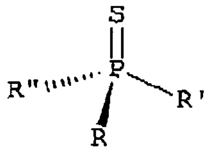

I II

bereit, wobei

R, R' und R" verschieden sind und H, OH, SH, $NH_2$, Alkyl, Aryl, Alkoholat, Phenolat, Thiolat, Thiophenolat, primäres Amin, sekundäres Amin oder Halogenid bedeuten, oder die Reste R und R" zusammen ein substituiertes oder unsubstituiertes aliphatisches oder aromatisches, homozyklisches oder heterozyklisches Ringsystem bilden,
dadurch gekennzeichnet, dass

a) im ersten Reaktionsschritt ein chirales Auxiliar der Formel (VI)

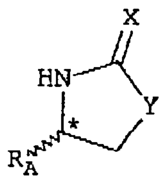

VI,

wobei
X NH, NCN, O oder S bedeutet,
Y NH, N($R^1$), O oder S bedeutet, wobei $R^1$ H, Alkyl oder Aryl ist,
$R_A$ Alkyl oder Aryl bedeutet und
* für R- oder S-Konfiguration steht,
am Phosphoratom von Phosphorylchlorid oder Thiophosphorylchlorid kovalent gebunden wird,

b) im Folgeschritt ein Alkohol, Thiol oder Amin als Nucleophil (Nu1) in Gegenwart einer Base mit dem Produkt aus dem ersten Reaktionsschritt in Reaktion gebracht wird, und

c) im letzten Schritt das chirale Auxiliar (VI) durch ein Nucleophil (Nu2) verdrängt wird.

Die hier verwendeten Begriffe werden gemäß ihrer fachüblichen Bedeutung verwendet, sofern nicht ausdrücklich etwas anderes angegeben ist oder sich aus dem Zusammenhang nicht eindeutig etwas anderes ergibt.

Die Begriffe "stereoselektiv" oder "stereospezifisch" bedeuten in Zusammenhang mit dem erfindungsgemäßen Verfahren beispielsweise, dass bei der Herstellung einer Verbindung, die in verschiedenen stereoisomeren Formen auftritt, ein Stereoisomer bevorzugt und damit im Überschuss gebildet wird. Von einer "stereoselektiven" oder "stereospezifischen" Reaktion wird dann gesprochen, wenn die Reaktion zu einem prozentualen Stereomerenüberschuss von >0% führt. Von einem reinen Stereoisomer wird hier gesprochen, wenn der prozentuale Stereomerenüberschuss >95% beträgt.

Unter einem "Stereoisomer" ("Stereomer") wird eine Verbindung verstanden, die im Vergleich mit einer anderen Verbindung dieselbe Konstitution, d.h. dieselbe Summenformel und Verknüpfung der Atome (Struktur), jedoch eine andere Konfiguration oder Konformation, d.h. räumliche Anordnung der Atome, aufweist. Im Falle von Konfigurationsisomeren ist eine Umwandlung der Isomere in das jeweils andere nur durch das Lösen und Neuknüpfen von kovalenten Bindungen möglich, im Falle von Konformationsisomeren (Konformeren) kann eine Überführung des einen Isomers in das andere durch freie Drehung um Einfachbindungen erfolgen. Zur Kennzeichnung der Konfiguration von Stereoisomeren bzw. Atomen innerhalb von Stereoisomeren wird allgemein die R-S-Nomenklatur gemäß dem Cahn-Ingold-Prelog-System verwendet. Die Buchstaben "R" (vom lateinischen "rectus") und "S" (vom lateinischen "sinister") kennzeichnen dabei die jeweiligen Isomere. Je nachdem, ob sich die Angabe auf ein Kohlenstoff oder beispielsweise Phosphoratom bezieht, kann jeweils ein Index angefügt werden. "$R_c$" bezeichnet somit die R-Konfiguration am C-Atom, "Sp" die S-Konfiguration am P-Atom.

Unter "Enantiomeren" werden Stereoisomere, verstanden, die sich wie Bild und Spiegelbild verhalten und keine Symmetrieebene innerhalb des Moleküls aufweisen. Dies gilt für entsprechende Konformations- und Konfigurationsisomere (Konformations- und Konfigurationsenantiomere). Konfigurationsenantiomere unterscheiden sich in allen Stereozentren und sind immer chiral. In der vorliegenden Anmeldung wird der Begriff "Enantiomer", sofern nicht ausdrücklich etwas anderes angegeben ist oder sich aus dem Zusammenhang für den Fachmann nicht etwas anderes ergibt, synonym zu Konfigurationsenantiomeren verwendet.

"Diastereomere" (Diastereoisomere) sind Stereoisomere, die keine Enantiomere sind. Diastereomere verhalten sich nicht wie Bild und Spiegelbild und können entweder chiral oder a-chiral sein. Auch bei Diastereomeren können Konformations-und Konfigurationsdiastereomere unterschieden werden. Konfigurationsdiastereomere mit mehreren Stereozentren unterscheiden sich voneinander in mindestens einem Stereozentrum, jedoch nicht in allen Stereozentren. In der vorliegenden Anmeldung wird der Begriff "Diastereomer", sofern nicht ausdrücklich etwas anderes angegeben ist oder sich aus dem Zusammenhang für den Fachmann nicht etwas anderes ergibt, synonym zu Konfigurationsdiastereomeren verwendet.

Als "Stereozentrum" bezeichnet man Atome, die unterschiedliche Substituenten tragen. Kohlenstoff, Stickstoff und Phosphor können beispielsweise Stereozentren ausbilden. Im Falle von Kohlenstoff beispielsweise spricht man von einem Stereozentrum, wenn das Kohlenstoffatom vier unterschiedliche Substituenten besitzt. Bei Phosphor beispielsweise kann auch ein Stereozentrum vorhanden sein, wenn das Phosphoratom drei unterschiedliche Substituenten und ein nichtbindendes Elektronenpaar aufweist. Stereozentren werden häufig mit "*" gekennzeichnet.

Mit dem Begriff "chiral" werden hier Moleküle bezeichnet, die mit ihrem Spiegelbild nicht deckungsgleich sind, d.h. nicht zur Deckung gebracht werden können. Chirale Moleküle weisen häufig, jedoch nicht notwendig, mindestens ein Stereozentrum auf.

Unter dem Begriff "Racemat" wird hier eine Mischung aus gleichen Anteilen von Stereomeren, d.h. Enantiomeren oder Diastereomeren, verstanden.

Mit dem "Stereomerenüberschuss" wird das Mengen- bzw. Massenverhältnis eines Stereomers zu dem entsprechenden anderen Stereoisomer in einer Mischung aus beiden Stereomeren, z.B. als Ergebnis einer stereoselektiven Reaktion, angegeben. Der prozentuale Stereomerenüberschuss s.e. ("<u>s</u>tereomeric <u>e</u>xcess") beschreibt das prozentuale Produktverhältnis der Stereoisomere A und B gemäß der Formel

$$s.e. = \frac{|m(A) - m(B)|}{m(A) + m(B)} \bullet 100,$$

wobei m(A) die Masse des Stereoisomers A und m(B) die Masse des Stereoisomers B ist.

Wenn A und B Diastereomere sind, spricht man vom Diastereomerenüberschuss d.e. ("<u>d</u>iastereomeric <u>e</u>xcess"), der das Produktverhältnis der Diastereomere A und B bei einer diastereoselektiven Reaktion angibt. Wenn A und B Enantiomere sind, spricht man vom Enantiomerenüberschuss e.e., der das Produktverhältnis der Enantiomere A und B bei einer enantioselektiven Reaktion angibt. Die Angabe eines Diastereomerenüberschusses d.e. von 80 % bedeutet beispielsweise, dass die Mischung sich aus 90 % des einen Diastereomers und 10 % des anderen Diastereomers zusammensetzt. Die 80 % geben somit nur den überschießenden Anteil des einen Diastereomers an. Racemate haben demnach einen Stereomerenüberschuss von 0 %, bei nur einem einzigen Stereoisomer ergibt sich ein Stereomerenüberschuss von 100 %.

Unter einem "Auxiliar" wird hier eine kovalent an einem Molekül angebrachte Gruppe oder Verbindung verstanden, die eine bestimmte Reaktion ermöglicht, erleichtert oder deren stereochemischen Verlauf beeinflusst. Das Auxiliar kann nach erfolgreicher Reaktion in einem weiteren Schritt wieder abgespalten werden. Unter einem "chiralen Auxiliar" wird eine Verbindung oder Gruppe verstanden, die chiral ist und als Auxiliar fungiert. Mit Hilfe eines chiralen Auxiliars kann der Verlauf einer gegebenenfalls nicht stereoselektiven Reaktion so gesteuert werden, dass nach Abspaltung des Auxiliars dennoch bevorzugt ein Stereoisomer erhalten wird.

Unter "Atomökonomie" oder "Atomeffizienz" wird der prozentuale Anteil der in einer chemischen Reaktion von den Edukten in die Produkte überführten Atome verstanden.

Der Begriff "Nucleophil" ist dem Fachmann bekannt und hat hier die dem Fachmann geläufige Bedeutung. Insbesondere wird hier unter einem Nucleophil ein Molekül verstanden, das einen negativ polarisierten Bereich, eine negativ polarisierte funktionelle Gruppe oder ein freies Elektronenpaar, in der Regel in einem energiereichen Orbital, enthält. Dabei umfasst der Begriff auch Moleküle, die im Verhältnis zu einem betrachteten Reaktionspartner bzw. zu einem Bereich des Reaktionspartners nucleophil, d.h. verhältnismäßig elektronenreicher sind. Der Reaktionspartner wird auch als Elektrophil bezeichnet, weil er Elektronen vom Nucleophil übernimmt. Nucleophile können kovalente Bindungen ausbilden, indem sie einem Reaktionspartner Elektronen zur Verfügung stellen. Dabei stammen die für die Bindung benötigten Elektronen regelmäßig allein vom Nucleophil. Nucleophile können negativ geladen (Anionen) sein. Beispiele für typische nucleophile Reagenzien sind Carbanionen, Anionen, Lewis-Basen, Aromaten, Alkohole, Amine, und Verbindungen mit olefinischen Doppelbindungen. Die Stärke der Nucleophilie hängt beispielsweise vom Reaktionspartner, der Basizität, dem Lösungsmittel und sterischen Faktoren ab. Dem Fachmann sind die Faktoren, die die Nucleophilie einer Verbindung beeinflussen, gut bekannt und er kann daher leicht deren nucleophile Eigenschaften bestimmen. Die Nucleophilie eines Moleküls wird dabei vorteilhaft auf das am stärksten nucleophile Atom bzw. die am

stärksten nucleophile funktionelle Gruppe bezogen. Sie kann aber ebenso auf ein ausgewähltes Atom oder eine ausgewählte Gruppe bezogen werden, die eine bestimmte Reaktion mit einer Verbindung eingehen soll.

Unter einem "Nucleosid" werden hier organische Moleküle verstanden, die aus einem Zuckerrest und einer organischen Base, z.B. einer heterocyclischen organischen Base, insbesondere einer stickstoffhaltigen heterocyclischen organischen Base, bestehen. Der Zuckerrest ist in der Regel eine Pentose, z.B. Desoxyribose oder Ribose, kann aber auch ein anderer Zucker sein. Unter einer "Nucleobase werden" organische Basen verstanden, die in RNA oder DNA vorkommen. Bei den Nucleobasen handelt es sich häufig um Purine (R) und Pyrimidine (Y). Beispiele für Purine sind Guanin (G) und Adenin (A), Beispiele für Pyrimidine sind Cytosin (C), Thymin (T) und Uracil (U). Phosphorylierte Nucleoside, beispielsweise Nucleosidmonophosphate (NMP), Nucleosiddiphosphate (NDP) und Nucleosidtriphosphate (NTP), werden auch als Nucleotide bezeichnet. Die Phosphat-, Diphosphat- (Pyrophosphat-) bzw. Triphosphatgruppe ist in der Regel mit dem 5'-C-Atom der Zuckerkomponente des Nucleosids verknüpft, kann aber beispielsweise auch mit dem 3'-C-Atom verknüpft sein.

Unter einem "Nucleosidanalogon" wird hier eine Verbindung verstanden, die im menschlichen Körper natürlicherweise nicht vorkommt, einem natürlicherweise im menschlichen Körper vorkommenden Nucleosid aber strukturell so ähnlich ist, dass es beispielsweise von der Zelle und/oder von viralen Enzymen im Wesentlichen entsprechend dem natürlichen Nucleosid verarbeitet, beispielsweise phosphoryliert und in einen RNA- oder DNA-Strang eingebaut wird. Ein Nucleosidanalogon kann selbst ein Nucleosid sein. Es kann aber beispielsweise auch eine andere Verbindung mit den obigen Eigenschaften sein, beispielsweise eine Verbindung aus einer heterocyclischen Base und einem Rest, der kein Zucker ist. Beispiele für Nucleosidanaloga sind beispielsweise AZT (3'-Azido-2',3'-didesoxythimidin, Azidothymidin), 2',3'-Didesoxyinosin (Didanosin), 2',3'-Didesoxycytidin (Zalticabin) und 2-Amino-9-((2-hydroxy-ethoxy)methyl)-1H-purin-6(9H)-on (Acyclovir). Auch Nucleosidphosphonate können Nucleosidanaloga sein.

Der Begriff "Alkyl" beinhaltet gesättigte aliphatische Gruppen, einschließlich geradkettiger Alkylgruppen (z.B. Methyl, Ethyl, Propyl, Butyl, Pentyl, Hexyl, Heptyl und Octyl), verzweigtkettiger Alkylgruppen (z.B. Isopropyl, tert-Butyl, Isobutyl), Cycloalkyl- (z.B. alizyklische) Gruppen (z.B. Cyclopropyl, Cyclopentyl, Cyclohexyl, Cycloheptyl, Cyclooctyl), alkylsubstituierte Cycloalkylgruppen und cycloalkylsubstituierter Alkylgruppen. "Alkyl" beinhaltet ferner Alkylgruppen, die Sauerstoff-, Stickstoff-, Schwefel- oder Phosphoratom aufweisen, die ein oder mehrere Kohlenstoffatome des Kohlenwasserstoffgerüsts ersetzen. Der Begriff "Alkyl" beinhaltet ebenfalls sowohl unsubstituierte Alkyle als auch substituierte Alkyle, wobei sich das letztere auf Alkylreste bezieht, die Substituenten aufweisen, die ein Wasserstoffatom an einem oder mehreren Kohlenstoffatomen des Kohlenwasserstoffgerüsts ersetzen. Solche Substituenten können zum Beispiel beinhalten: Alkyl, Alkenyl, Alkynyl, Halogen, Hydroxyl, Alkylcarbonyloxy, Arylcarbonyloxy, Alkoxycarbonyloxy, Aryloxycarbonyloxy, Carboxylat, Alkylcarbonyl, Arylcarbonyl, Alkoxycarbonyl, Aminocarbonyl, Alkylaminocarbonyl, Dialkylaminocarbonyl, Alkylthiocarbonyl, Alkoxyl, Phosphat, Phosphonato, Phosphinato, Cyano, Amino (einschließlich Alkylamino, Dialkylamino, Arylamino, Diarylamino und Alkylarylamino), Acylamino (einschließlich Alkylcarbonylamino, Arylcarbonylamino, Carbamoyl und U-reido), Amidino, Imino, Sulfhydryl, Alkylthio, Arylthio, Thiocarboxylat, Sulfate, Alkylsulfinyl, Sulfonato, Sulfamoyl, Sulfonamido, Nitro, Trifluoromethyl, Cyano, Azido, Heterocyclyl, Alkylaryl oder ein aromatischer oder heteroaromatischer Rest. Cycloalkyle können weiter substituiert sein, z.B. mit den oben angegebenen Substituenten. Ein "Alkylaryl"- oder ein "Aralkyl"-Rest ist ein Alkyl, das mit einem Aryl substituiert ist (z.B. Phenylmethyl (Benzyl)). "Alkyl" beinhaltet auch die Seitenketten von natürlichen und unnatürlichen Aminosäuren.

Unter "Aryl" werden Gruppen mit Aromatizität verstanden, einschließlich 5- und 6-gliedrigen aromatischen Einzelringgruppen, die null bis vier Heteroatome beinhalten können, sowie multizyklischen Systemen mit mindestens einem aromatischen Ring. Beispiele für Aryl-Gruppen beinhalten Benzol, Phenyl, Pyrrol, Furan, Thiophen, Thiazol, Isothiazol, Imidazol, Triazol, Tetrazol, Pyrazol, Oxazol, Isooxazol, Pyridin, Pyrazin, Pyridazin und Pyrimidin, und dergleichen. Darüber hinaus beinhaltet der Begriff "Aryl" multizyklische Aryl-Gruppen, z.B. trizyklische, bizyklische, z.B. Naphthalen, Benzoxazol, Benzodioxazol, Benzothiazol, Benzoimidazol, Benzothiophen, Methylenedioxyphenyl, Quinolin, Isoquinolin, Napthridin, Indol, Benzofuran, Purin, Benzofuran, Deazapurin oder Indolizin. Unter "Aryl" werden auch Aryl-Gruppen verstanden, die Heteroatome in der Ringstruktur aufweisen ("Heteroaryle"). Der aromatische Ring kann an ein oder mehreren Ringpositionen substituiert sein. Aryl-Gruppen können auch mit alizyklischen oder heterocyclischen Ringen, die nicht aromatisch sind, fusioniert oder verbrückt sein, so dass ein multizyklisches System gebildet wird (z.B. Tetralin, Methylenedioxyphenyl).

Unter "Alkoholat" werden Alkoholreste R-O- verstanden. "Alkohole" sind Verbindungen gemäß der Formal R-OH. R kann dabei ein beliebiger organischer Rest, d.h. ein von einer organischen Verbindung gebildeter Rest, gegebenenfalls mit weiteren Hydroxygruppen, beispielsweise ein Alkyl- oder Arylrest sein. Unter "Phenolat" wird eine aromatische Hydroxyverbindung verstanden, deren OH-Gruppe(n) am aromatischen Kern sitzt bzw. sitzen, z.B. $C_6H_5$-O-. "Thiolate" sind Verbindungen der Formel R-S-, wobei R ein beliebiger organischer Rest, beispielsweise ein Alkyl- oder Arylrest sein kann. Thiolate entsprechen Alkoholaten, wobei der Sauerstoff der Hydroxygruppe durch Schwefel ersetzt ist. "Thiophenolate" sind Phenolate gemäß der obigen Definition, mit der Maßgabe, dass der Sauerstoff zumindest einer Hydroxygruppe durch Schwefel ersetzt ist. "Thiole" sind Verbindungen der Formel R-SH, gegebenenfalls mit weiteren SH-Gruppen, wobei R ein beliebiger organischer Rest, beispielsweise ein Alkyl- oder Arylrest sein kann.

"Amine" sind Derivate des Ammoniaks ($NH_3$), wobei eines oder mehrere der Wasserstoffatome des Ammoniaks durch organische Reste, z.B. Alkyl- oder Arylreste, ersetzt sind. "Primäre Amine" sind Amine, bei denen nur eines der Wasserstoffatome durch einen organischen Rest, z.B. einen Alkyl- oder Arylrest, ersetzt ist. "Sekundäre Amine" sind Amine, bei denen zwei der Wasserstoffatome durch einen organischen Rest, z.B. einen Alkyl- oder Arylrest, ersetzt ist.

Unter einem "Halogenid" werden Halogenreste, z.B. F-, C1-, Br-, I- und At-, verstanden.

Der Begriff "Base" ist dem Fachmann ebenfalls geläufig und umfasst Verbindungen oder Substanzen, die ein freies Elektronenpaar besitzen, mit dem es zu einem Atom, Molekül oder Ion eine kovalente Bindung ausbilden kann (Elektronenpaarakzeptor, Lewis-Base). Der Begriff umfasst auch ein Molekül oder Ion, das über ein freies Elektronenpaar verfügt, mit dem es Protonen anziehen und festhalten kann (Protonenakzeptor, Brönsted-Base). Der Begriff umfasst darüber hinaus auch Substanzen, die zur Bildung des für das jeweilige Lösungsmittel charakteristischen Anions, in wässriger Lösung beispielsweise zum Hydroxidion, führen und in Reaktion mit der entsprechenden Säure durch Neutralisation das Lösungsmittel, z.B. Wassern, bilden (Arrhenius-Base).

Das erfindungsgemäße Verfahren zur stereoselektiven Synthese von Phosphorverbindungen, insbesondere Organophosphorverbindungen wie Phosphortriester, beruht auf dem Einsatz eines chiralen Auxiliars gemäß der folgenden Formel VI

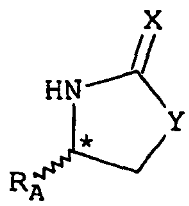

VI,

wobei

X NH, NCN, O oder S bedeutet,

Y NH, $N(R^1)$, O oder S bedeutet, wobei $R^1$ H, Alkyl oder Aryl sein kann,

$R_A$ Alkyl oder Aryl bedeutet und

* für R- oder S-Konfiguration steht.

Das chirale Auxiliar gemäß Formel VI, das in der $R_c$- oder $S_c$-Konfiguration vorliegen kann, führt zu einer hohen Induktion am Phosphoratom und kann unter milden Reaktionsbedingungen substituiert werden.

Mit "Induktion" ist hier der dem Fachmann bekannte so genannte induktive Effekt (I-Effekt) gemeint, der einen ladungsverändernden, "elektronenziehenden" oder "elektronenschiebenden", Effekt einer funktionellen Gruppe oder eines Atoms bezeichnet. Der induktive Effekt beruht auf Elektronegativitätsunterschieden zwischen Atomen oder funktionellen Gruppen eines Moleküls, was zur Polarisation von Atombindungen.führt.

Ein Beispiel für ein unter die obige Formel VI fallendes Auxiliar ist $S_c$-4-iso-Propyl-2-mercapto-2-thiazolin 1

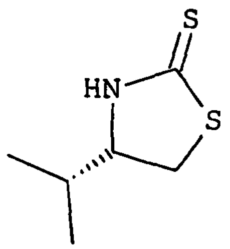

1

Andere Derivate der Verbindung VI können vom Fachmann leicht hergestellt und gegebenenfalls für den gewünschten Einsatzzweck gezielt gewählt oder ausgestaltet werden. Die Reaktionsbedingungen, unter denen das chirale Auxiliar VI mit Phosphorylchlorid oder Thiophosphorylchlorid 2 zum entsprechenden Reaktionsprodukt 10, das im folgenden auch als Aktivester bezeichnet wird, umgesetzt wird, können vom Fachmann hinsichtlich Lösungsmittel, eingesetzter Base, Reaktionstemperatur und Reaktionszeit ohne Weiteres gegebenenfalls optimiert werden, ohne das hierzu mehr als nur Routineversuche erforderliche wären.

Im Folgeschritt können beliebige Alkohole, Thiole oder Amine als Nucleophil (Nu1) eingesetzt werden, so dass die entsprechenden Ester 11, Thioester 12, Amidate bzw. Diamidate 13 gebildet werden, wie im Folgenden beispielhaft dargestellt ist:

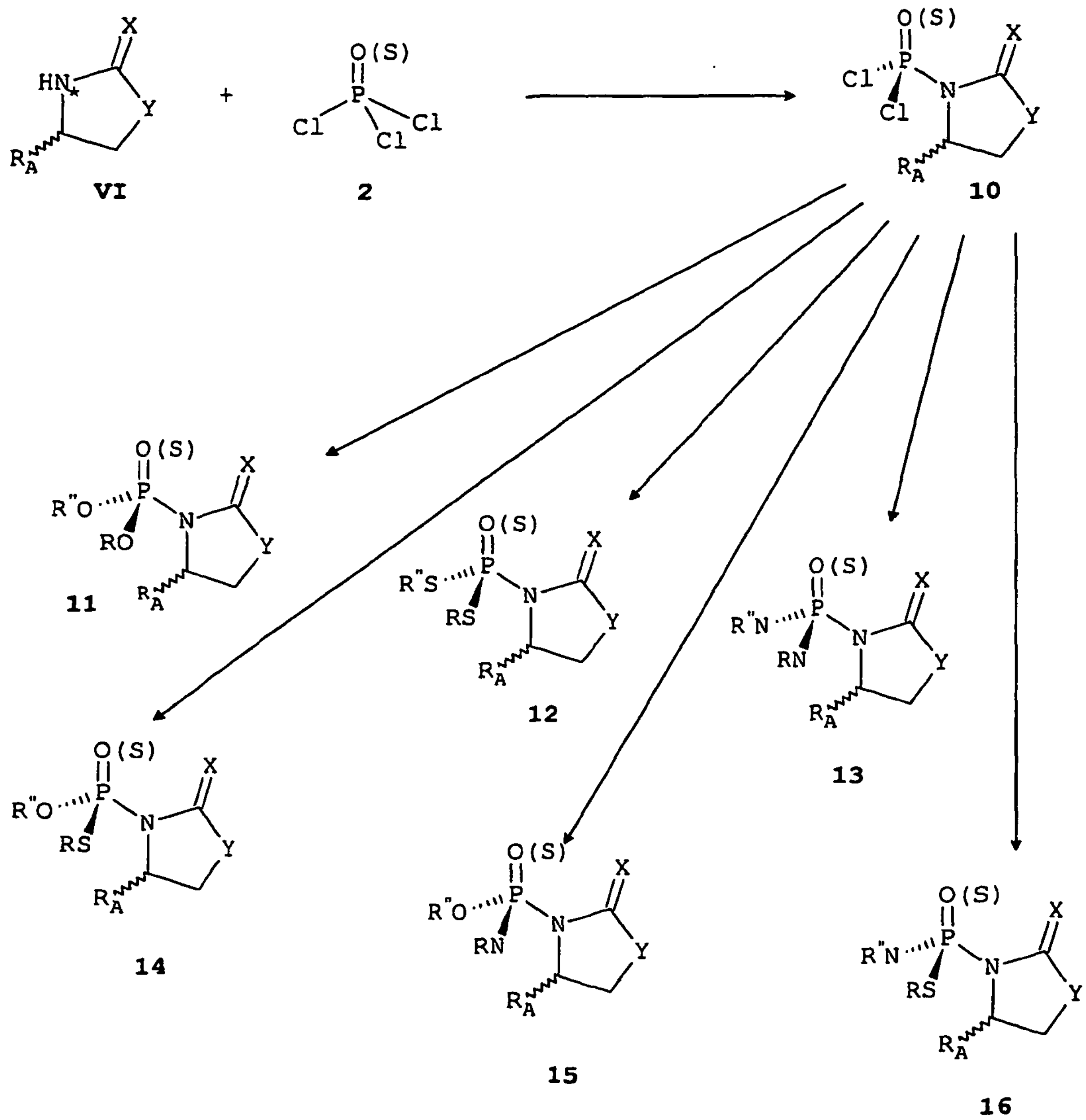

VI 2 10

11 12 13 14 15 16

Darüber hinaus lassen sich auch die gemischten Verbindungen 14-16 darstellen. Wichtig bei dieser Reaktion ist, dass neben dem Nucleophil (Nu1) auch eine Base anwesend ist. Dabei kann je nach Alkohol, Thiol bzw. Amin durch die unterschiedliche Stärke der verwendeten Base die Ausbeute der Reaktion beeinflusst werden. Wichtig ist bei den oben dargestellten Reaktionen insgesamt, dass durch das am Phosphoratom kovalent angeknüpfte chirale Auxiliar VI auch bei diesen Reaktionen eine Induktion beobachtet wird, so dass die Verbindungen 11-16 sämtlich chiral bezüglich der Stereochemie am P-Atom sind. Selbstverständlich lassen sich aber auch achirale Phosphatderivate auf diesem Wege darstellen. Ein im Folgeschritt besonders bevorzugtes Nucleophil (Nu1) ist Salicylalkohol (2-Hydroxybenzylalkohol).

Im letzten Schritt werden die als chirale Auxiliare VI eingesetzten Fünfring-Heterocyclen durch ein Nucleophil (Nu2) verdrängt. Dabei findet eine Inversion der Konfiguration am Phosphoratom statt, so dass insgesamt chirale Phosphate entstehen, sofern das Produkt aus Aktivester und im Folgeschritt verwendetem Nucleophil (Nu2) ebenfalls chiral ist. Dabei sind die Reaktionsbedingungen den eingesetzten Auxiliaren anzupassen, was dem Fachmann jedoch anhand seines Fachkönnens, gegebenenfalls nach Durchführung von Routineversuchen, ohne Weiteres möglich ist. Durch eine zusätzliche Metallsalz-Aktivierung lassen sich die Austrittsgruppeneigenschaften gezielt modifizieren. Beispielhaft sei hier die Verwendung von Quecksilbersalzen bei schwefelhaltigen Auxiliaren genannt. Kupfersalze eignen sich für sauerstoffhaltige Auxiliare.

Durch stufenweisen Austausch der zu Beginn der Reaktion am Phosphoratom gebundenen Chloratome können

folglich chirale Phosphorsäure-Derivate gebildet werden. Startet man am Anfang mit dem entsprechenden Thiophosphorylchlorid, lässt sich somit auch eine Vielzahl von chiralen Thiophosphorsäure-Verbindungen synthetisieren.

Mit Hilfe des erfindungsgemäßen Verfahrens lassen sich die Verbindungen I-II

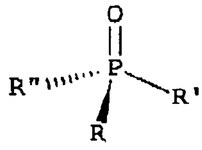
I

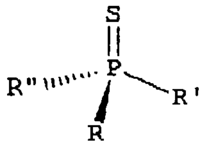
II

synthetisieren, wobei die Reste R, R' und R" beispielsweise auch unterschiedliche Isotope eines Elements sein oder enthalten können.

Im Folgenden ist beispielhaft eine Reihe von Zielverbindungen dargestellt, die sich durch das neue Verfahren synthetisieren lassen (Nucl = Nucleosid). Darunter sind z.B. für eine Anwendung als Pharmaka ausgesprochen interessante Verbindungen (z.B. 18 oder 19). Dies betrifft den Bereich der Nucleotid-Prodrugs (Propharmaka) genauso wie den Bereich Antisense-Oligonucleotide. Außerdem kann das Verfahren auch für die Synthese von chiralen Phosphor (III) Verbindungen eingesetzt werden. Solche chiralen Verbindungen sind als Liganden bei metallkatalysierten Reaktionen extrem interessant.

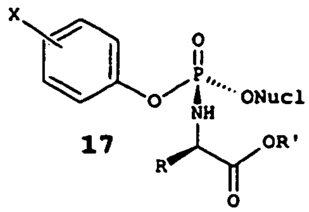
17

X=Elektronenakzeptor
R=Alkyl; R'=Alkyl,Aryl

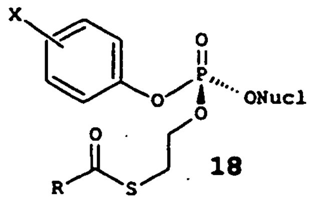
18

X=Elektronenakzeptor
R=Alkyl; R'=Alkyl,Aryl

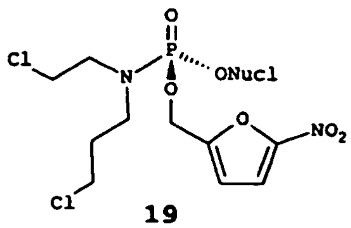
19

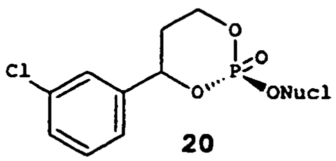
20

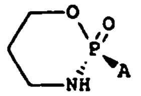
Cyclophosphamide
21

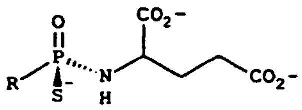
22

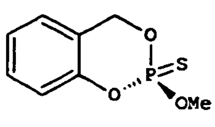

Insektizide
**23**

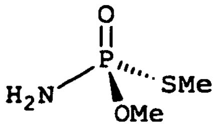

**24**

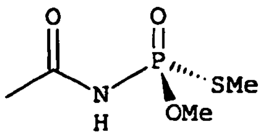

**25**

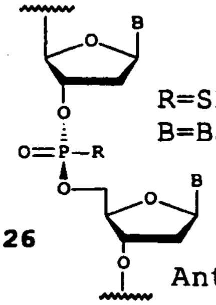

R=SH, Alkyl
B=Base

**26**

Antisense

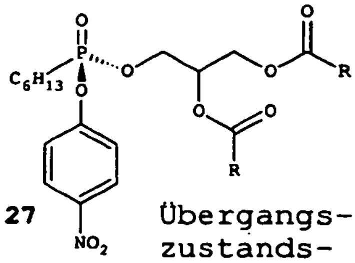

**27** Übergangs-zustands-analoga

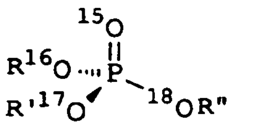

R≠R'≠R"=H, Alkyl, Aryl

**28**

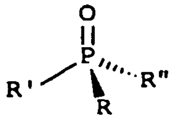

**29**

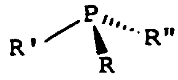

**30**

R≠R'≠R"=H, Alkyl, Aryl
cyklische, acyclische P-chirale Liganden

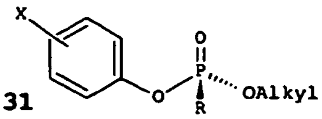

**31**

Lipaseinhibitor
R=Alkyl, Aryl, Glycerolreste

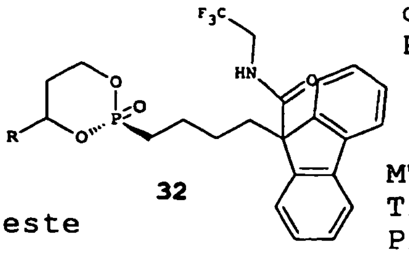

**32**

MTP = Mikrosomales Triglycerid-Transfer-Protein

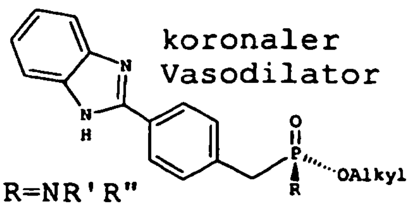

koronaler Vasodilator

R=NR'R"

**33**

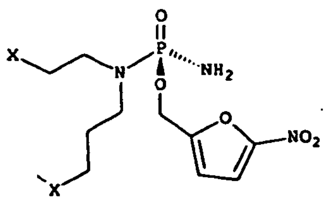

**34**

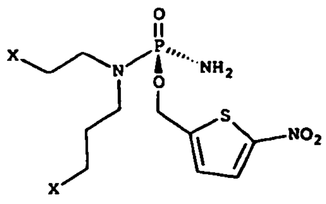

**35**

X = H, Alkyl, Aryl, Halogenid

Durch das erfindungsgemäße Verfahren können beispielsweise die folgenden chiralen, nicht-racemischen Phosphorverbindungen dargestellt werden:

1. Derivate der Phosphorsäure (Ester, Amidate)
2. Derivate der Phosphonsäure (Ester, Amidate)
3. Derivate der Phosphinsäure (Ester, Amidate)
4. Derivate der Thio-Phosphorsäure (Ester, Amidate)
5. Derivate der Thio-Phosphonzäure (Ester, Amidate)
6. Derivate der Thio-Phosphinsäure (Ester, Amidate)
7. Phosphazane
8. Phosphazene
9. Halogenphosphorsauren (Ester, Amidate)
10. Dihalogenphosphorsäure (Ester, Amidate)
11. Phosphorylhalogenide (Ester, Amidate)
12. Halogenthiophosphorsäuren (Ester, Amidate)
13. Dihalogenthiohosphorsäure (Ester, Amidate)
14. Thiophosphorylhalogenide
15. Phosphite, Phosphine, Phosphoramidite
16. Pepsin- und Penicilopepsin-Inhibitoren, Peptid-Analoga
17. Aminophosphonate, Aminophosphinate, Lipase-Inhibitoren, Peptid-Mimetika, Antagonisten von Carbonsäurederivaten
18. Cyclophosphamide, P-chirale Phosphinliganden

Die für die Durchführung dieser Reaktionen benötigten Ausgangsmaterialien sind in der Regel günstig und leicht zugänglich. Darüber hinaus sollte es möglich sein, die Reaktionen auch in großem Maßstab durchzuführen.

In einer bevorzugten Ausführungsform wird bei dem erfindungsgemäßen Verfahren ein im Folgeschritt b) im Überschuss gebildetes Stereoisomer zunächst gereinigt und in dieser gereinigten Form im letzten Schritt c) eingesetzt. Auf diese Weise kann die unerwünschte Reaktion des Nucleophils (Nu2) mit dem im Unterschuss vorhandenen Stereoisomer weitgehend vermieden und die Atomökonomie der Reaktion weiter verbessert werden.

Das in Schritt c) eingesetzte Nucleophil (Nu2) kann beispielsweise ein Nucleophil sein, dass ausgewählt ist aus der Gruppe, bestehend aus Nucleosid, Nucleosidmonophosphat, Nucleosiddiphosphat, Nucleosidtriphosphat, Nucleosidanalogon, Nucleosidmonophosphatanalogon, Nucleosiddiphosphatanalogon, Nucleosidtriphosphatanalogon, Phosphat, Pyrophosphat, Glycosylphosphat und $\alpha$-deprotoniertem Glykosyl, oder Salzen davon.

In einer besonders bevorzugten Ausführungsform des erfindungsgemäßen Verfahrens wird 4-iso-Propyl-2-mercapto-2-thiazolin als chirales Auxiliar (VI) verwendet und unter Bildung von ($S_c$)-N-Dichlorphosphoryl-4-iso-propyl-2-mercapto-2-thiazolin kovalent an das Phosphoratom von Phosphorylchlorid gebunden, das ($S_c$)-N-Dichlorphosphoryl-4-iso-propyl-2-mercapto-2-thiazolin wird im Folgeschritt b) mit Salicylalkohol in Reaktion gebracht, die entstandenen ($R_p$,$S_c$)- und ($S_p$,$S_c$)-Diastereomere werden getrennt und das chirale Auxiliar (VI) wird anschließend durch ein Nucleosid unter Bildung von cycloSaligenyl-Nucleosidmonophosphat aus dem ($R_p$,$S_c$)-Diastereomer verdrängt.

Mit dem erfindungsgemäßen Verfahren ist es erstmalig möglich, einen synthetischen Zugang zu diastereomerenreinen cycloSal-NMPs zu eröffnen. Das erfindungsgemäße Verfahren besticht durch die kurze Synthesesequenz und ihre potentielle Anwendbarkeit für viele cycloSal-NMPs. Damit ermöglicht also das erfindungsgemäße Verfahren die isomerenreine Darstellung von cycloSalNucleotid-Prodrugs. Diese können ausgesprochen interessante Eigenschaften hinsichtlich einer z.B. antiviralen Aktivität aufweisen und sind von großem Interesse für eine pharmazeutische Anwendung.

Ein als Nucleophil (Nu2) in Schritt c) eingesetztes Nucleosid ist vorzugsweise ausgewählt aus der Gruppe bestehend aus Adenosin, Guanosin, Cytidin, Thymidin, Uridin, Desoxyadenosin, Desoxyguanosin, Inosin, Desoxycytidin, Desoxyuridin, Desoxythymidin, 2-Thiocytidin, $N^4$-Acetyl-cytidin, 2'-O-methyl-cytidin, 3-Methyl-cytidin, 5-Methyl-cytidin, 2-Thiouridin, Pseudouridin, Dihydrouridin, 5-(Carboxyhydroxymethyl)-Uridin, 5-Carboxymethylaminomethyl-Uridin, 5-Methylaminomethyl-Uridin, 5-Methoxy-carbonylmethyl-Uridin, 5-Methoxy-Uridin, Ribo-Thymidin, 1-Methyl-adenosin, 2-Methyl-adenosin, $N^6$-Methyl-adenosin, Inosin, 1-Methyl-inosin, Guanosin, $N^2$-2,2-Dimethyl-guanosin, $N^2$-2-Methyl-guanosin, $7^+$-Methyl-guanosin und 2'-O-Methyl-guanosin.

Besonders bevorzugt beträgt der prozentuale Stereomerenüberschuss bei dem erfindungsgemäßen Verfahren nach Folgeschritt b) ≥10%, bevorzugt ≥20%, weiter bevorzugt ≥30%, ≥40%, ≥50% oder ≥60%, und besonders bevorzugt ≥70%, ≥75%, ≥80%, ≥85% oder ≥90%.

Eine anschließend vorgenommene Reinigung, beispielsweise chromatographische Reinigung, führt vorzugsweise zu einem prozentualen Stereomerenüberschuss von ≥95%, bevorzugt ≥96%, weiter bevorzugt ≥97% oder ≥98%, besonders bevorzugt ≥99%, ≥99,5%, ≥99,6%, ≥99,7%, ≥99,8% oder ≥99,9%.

Die Erfindung wird im Folgenden anhand eines Ausführungsbeispiels zu Veranschaulichungszwecken näher beschrieben.

Beispiel 1: Diastereoselektive Darstellung stereoisomerenreiner cycloSal-NMPs

Die stereoselektive Synthese eines stereoisomerenreinen cycloSal-NMPs (cycloSaligenyl-Nucleosidmonophosphats) ist im Folgenden schematisch dargestellt:

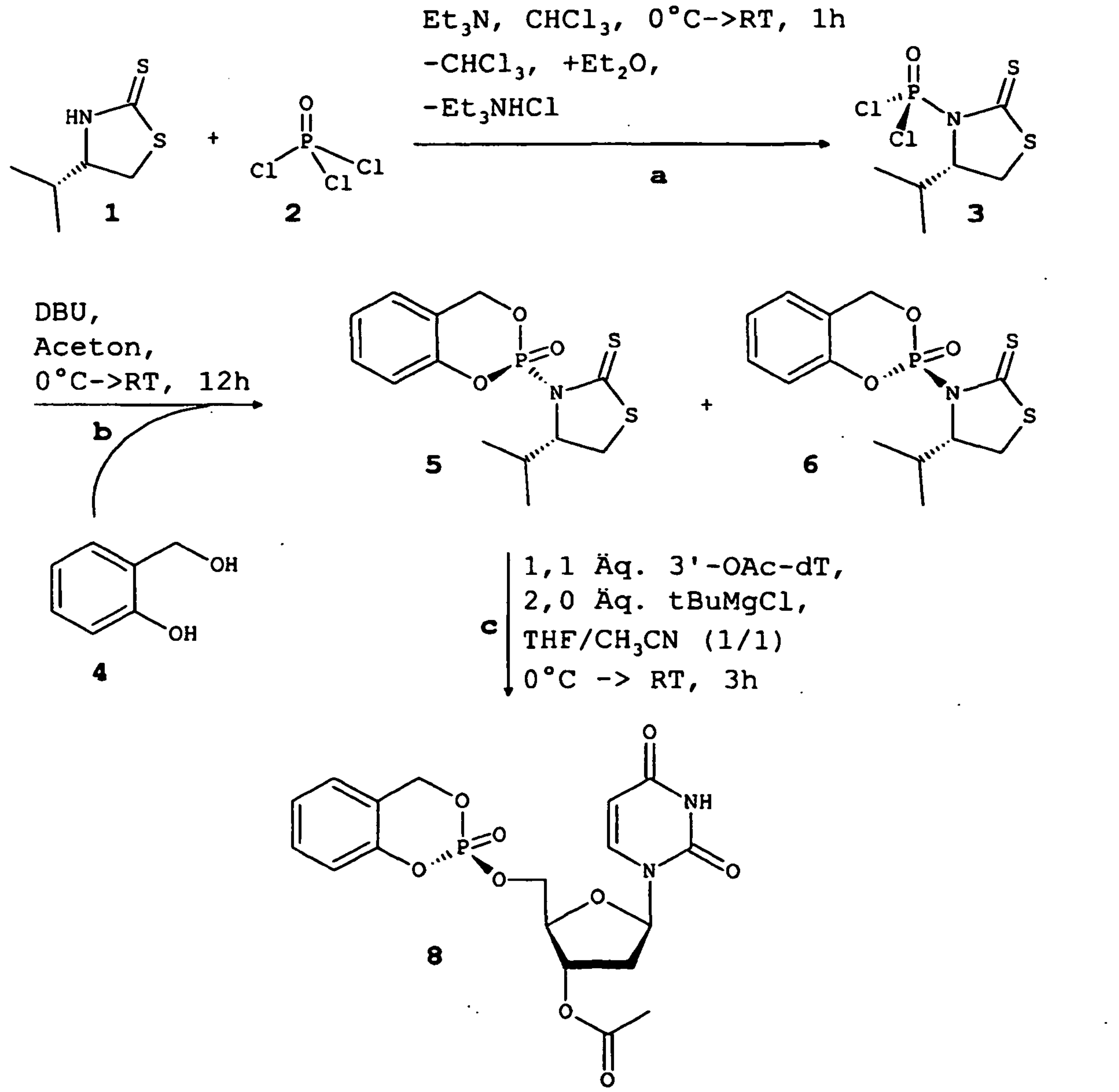


Der erfindungsgemäße Ansatz zur diastereoselektiven Synthese von cycloSal-NMPs beruht auf dem Einsatz des chiralen Auxiliars $S_c$-4-iso-Propyl-2-mercapto-2-thiazolin 1, das zu einer hohen Induktion am Phosphoratom führt und unter milden Reaktionsbedingungen substituiert werden kann, so dass in einer dreistufigen Reaktionssequenz isomerenreine cycloSal-NMPs erhalten werden können.

Dazu wird das chirale Auxiliar 1 mit Phosphorylchlorid 2 im Schritt a) zum ($S_c$)-N-Dichlorphosphoryl-4-isopropyl-2-mercapto-2-thiazolin 3 umgesetzt. Dieses geschieht in Chloroform mit Triethylamin als Protonenfänger bei 0 °C in nahezu quantitativer Ausbeute. Aufgrund der hohen Reaktivität des Produktes 3 wird unter inerten Reaktionsbedingungen entstandenes Triethylammoniumhydrochlorid filtriert und das Produkt 3 anschließend sofort umgesetzt. Die Kupplung des Produktes 3 mit Salicylalkohol 4 in Schritt b) erfolgte in einer 43%igen Ausbeute. Der Diastereomerenüberschuss d.e. betrug 88% und die Diastereomere 5 ($R_P$, $S_C$) und 6 ($S_P$, $S_C$) konnten durch chromatographische Reinigung als reine Isomere erhalten werden (>95% d.e., bestimmt durch NMR-Integration). Dazu wurde 3 mit 4 in Aceton gelöst und mit einer Lösung von 1,8-Diazabicyclo[5.4.0]undec-7-en (DBU) in Aceton bei 0 °C versetzt und 12 Stunden bei Raumtemperatur gerührt. Die anschließende Substitution des chiralen Auxiliars aus dem Aktivimid 5 durch das Nucleosid 3'-O-Acetyl-thymidin führte in einer 48%igen Ausbeute zum diastereomerenreinen $S_P$-cycloSal-NMP 8. Dazu wurde das Nucleosid 3'-O-Acetyl-thymidin in einem THF/Acetonitril-Gemisch (1/1) gelöst und mit der Base tert-Butylmagnesiumchlorid (t-BuMgCl) deprotoniert. Die entstandene Suspension wurde langsam zum ($R_P$,$S_C$)-Aktivimid 5 (gelöst in einem THF/Acetonitril-Gemisch, 1/1) gegeben. Nach drei Stunden wurde die Reaktion abgebrochen und das Produkt 8 chromatographisch gereinigt.

**Patentansprüche**

1. Verfahren zur stereoselektiven Synthese von Phosphorverbindungen der Formel I oder II

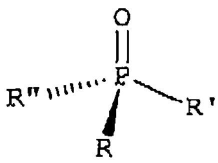
I

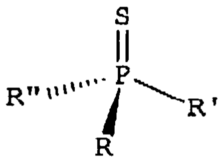
II

wobei
R, R' und R" verschieden sind und H, OH, SH, $NE_2$, Alkyl, Aryl, Alkoholat, Phenolat, Thiolat, Thiophenolat, primäres Amin, sekundäres Amin oder Halogenid bedeuten, oder die Reste R und R" zusammen ein substituiertes oder unsubstituiertes aliphatisches oder aromatisches, homozyklisches oder heterozyklisches Ringsystem bilden,
**dadurch gekennzeichnet, dass**

a) im ersten Reaktionsschritt ein chirales Auxiliar der Formel (VI)

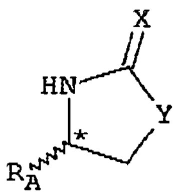
VI,

wobei
X NH, NCN, O oder S bedeutet,
Y NH, N($R^1$), O oder S bedeutet, wobei $R^1$ H, Alkyl oder Aryl ist,
$R_A$ Alkyl oder Aryl bedeutet und
* für R- oder S-Konfiguration steht,
am Phosphoratom von Phosphorylchlorid oder Thiophosphorylchlorid kovalent gebunden wird,
b) im Folgeschritt ein Alkohol, Thiol oder Amin als Nucleophil (Nu1) in Gegenwart einer Base mit dem Produkt aus dem ersten Reaktionsschritt in Reaktion gebracht wird, und
c) im letzten Schritt das chirale Auxiliar (VI) durch ein Nucleophil (Nu2) verdrängt wird.

2. Verfahren nach Anspruch 1, **dadurch gekennzeichnet, dass** ein im Folgeschritt b) im Überschuss gebildetes Stereoisomer gereinigt und im letzten Schritt c) eingesetzt wird.

3. Verfahren nach Anspruch 1 oder 2, **dadurch gekennzeichnet, dass** das in Schritt c) eingesetzte Nucleophil (Nu2) ausgewählt ist aus der Gruppe, bestehend aus Nucleosid, Nucleosidmonophosphat, Nucleosiddiphosphat, Nucleosidtriphosphat, Nucleosidanalogon, Nucleosidmonophosphatanalogon, Nucleosiddiphosphatanalogon, Nucleosidtriphosphatanalogon, Phosphat, Pyrophosphat, Glycosylphosphat und $\alpha$-deprotoniertem Glykosyl, oder Salzen davon.

4. Verfahren Anspruch 3, **dadurch gekennzeichnet, dass** 4-isoPropyl-2-mercapto-2-thiazolin als chirales Auxiliar (VI) verwendet und unter Bildung von ($S_C$)-N-Dichlorphosphoryl-4-isopropyl-2-mercapto-2-thiazolin kovalent an das Phosphoratom von Phosphorylchlorid gebunden wird, das ($S_C$)-N-Dichlor-phosphoryl-4-iso-propyl-2-mercapto-2-thiazolin im Folgeschritt b) mit Salicylalkohol in Reaktion gebracht wird, die entstandenen ($R_P$,$S_C$)- und ($S_P$,$S_C$)-Diastereomere getrennt werden und das chirale Auxiliar (VI) anschließend durch ein Nucleosid unter Bildung von cycloSaligenyl-Nucleosidmonophosphat aus dem ($R_P$,$S_C$)-Diastereomer verdrängt wird.

5. Verfahren nach Anspruch 3 oder 4, **dadurch gekennzeichnet, dass** das Nucleosid ausgewählt ist aus der Gruppe

bestehend aus Adenosin, Guanosin, Cytidin, Thymidin, Uridin, Desoxyadenosin, Desoxyguanosin, Inosin, Desoxycytidin, Desoxyuridin, Desoxythymidin, 2-Thiocytidin, $N^4$-Acetyl-cytidin, 2'-O-methyl-cytidin, 3-Methyl-cytidin, 5-Methyl-cytidin, 2-Thiouridin, Pseudouridin, Dihydrouridin, 5-(Carboxyhydroxymethyl)-Uridin, 5-Carboxymethylaminomethyl-Uridin, 5-Methylaminomethyl-Uridin, 5-Methoxy-carbonylmethyl-Uridin, 5-Methoxy-Uridin, Ribo-Thymidin, 1-Methyl-adenosin, 2-Methyl-adenosin, $N^6$-Methyl-adenosin, Inosin, 1-Methyl-inosin, Guanosin, $N^2$-2,2-Dimethyl-guanosin, $N^2$-2-Methyl-guanosin, $7^+$-Methyl-guanosin und 2'-O-Methyl-guanosin.

6. Verfahren nach einem der vorhergehenden Ansprüche, **dadurch gekennzeichnet, dass** der prozentuale Stereomerenüberschuss nach Folgeschritt b) ≥10%, bevorzugt ≥20%, weiter bevorzugt ≥30%, ≥40%, ≥50% oder ≥60%, und besonders bevorzugt ≥70%, ≥75%, ≥80%, ≥85% oder ≥90% beträgt.

## Claims

1. Method for the stereoselective synthesis of phosphorus compounds of formula I or II

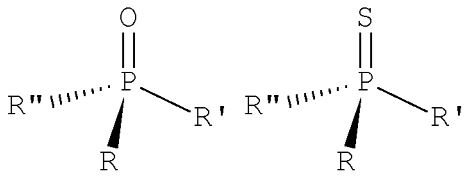

I II

wherein

R, R' and R" are different and denote H, OH, SH, $NH_2$, alkyl, aryl, alcoholate, phenolate, thiolate, thiophenolate, primary amine, secondary amine or halogenide, or the residues R and R" together form a substituted or unsubstituted aliphatic or aromatic, homocyclic or heterocyclic ring system, **characterized in that**

a) in the first reaction step a chiral auxiliary of formula (VI)

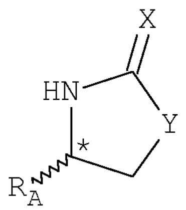

VI,

wherein

X denotes NH, NCN, O or S,

Y denotes NH, $N(R^1)$, O or S, wherein $R^1$ is H, alkyl or aryl, $R_A$ denotes alkyl or aryl and

* stands for R or S configuration,

is covalently bonded to the phosphorus atom of phosphoryl chloride or thiophosphoryl chloride,

b) in the subsequent step an alcohol, thiol or amine is reacted as a nucleophile (Nu1) with the product of the first reaction step in the presence of a base, and

c) in the last step the chiral auxiliary (VI) is displaced by a nucleophile (Nu2).

2. Method according to claim 1, **characterized in that** a stereoisomer formed in excess in the subsequent step b)is purified and employed in the last step c).

3. Method according to claim 1 or 2, **characterized in that** the nucleophile (Nu2) employed in step c) is selected from the group consisting of nucleoside, nucleoside monophosphate, nucleoside diphosphate, nucleoside triphosphate, nucleoside analog, nucleoside monophosphate analog, nucleoside diphosphate analog, nucleoside triphosphate

analog, phosphate, pyrophosphate, glycosyl phosphate and α-deprotonated glycosyl, or salts thereof.

**4.** Method according to claim 3, **characterized in that** 4-isopropyl-2-mercapto-2-thiazoline is used as chiral auxiliary (VI) and is covalently bonded to the phosphorus atom of phosphoryl chloride, forming ($S_C$)-N-dichlorophosphoryl-4-isopropyl-2-mercapto-2-thiazoline, ($S_C$)-N-dichlorophosphoryl-4-isopropyl-2-mercapto-2-thiazoline is reacted with salicyl alcohol in the subsequent step b), the resulting ($R_P$,$S_C$)- and ($S_P$,$S_C$)-diastereomers are separated and the chiral auxiliary (VI) is then displaced from the ($R_P$, $S_C$) diastereomer by a nucleoside, forming cycloSaligenyl nucleoside monophosphate.

**5.** Method according to claim 3 or 4, **characterized in that** the nucleoside is selected from the group consisting of adenosine, guanosine, cytidine, thymidine, uridine, deoxyadenosine, deoxyguanosine, inosine, deoxycytidine, deoxyuridine, deoxythymidine, 2-thiocytidine, $N^4$-acetylcytidine, 2'-O-methylcytidine, 3-methylcytidine, 5-methylcytidine, 2-thio-uridine, pseudouridine, dihydrouridine, 5-(carboxy-hydroxymethyl)-uridine, 5-carboxymethylaminomethyluridine, 5-methylaminomethyluridine, 5-methoxycarbonylmethyluridine, 5-methoxyuridine, ribothymidine, 1-methyladenosine, 2-methyladenosine, $N^6$-methyladenosine, inosine, 1-methylinosine, guanosine, $N^2$-2,2-dimethylguanosine, $N^2$-2-methylguanosine, $7^+$-methylguanosine and 2'-O-methylguanosine.

**6.** Method according to one of the preceding claims, **characterized in that** the percentage stereomeric excess after the subsequent step b) is ≥10%, preferably ≥20%, more preferably ≥30%, ≥40%, ≥50% or ≥60%, and especially preferably ≥70%, ≥75%, ≥80%, ≥85% or ≥90%.

## Revendications

**1.** Procédé de synthèse stéréosélective de composés phosphorés selon la formule I ou II

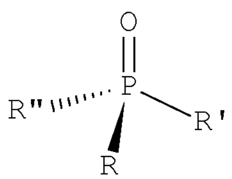
I

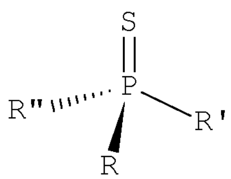
II

R, R' et R" étant différents et signifiant H, OH, SH, $NH_2$, de l'alkyle, de l'aryle, un alcoxyde alcoolate" un phénolate, un thiolate, un thiophénolate, une amine primaire, une amine secondaire ou de l'halogénure ou les restes R et R" formant ensemble une combinaison cyclique substituée ou non substituée, aliphatique ou aromatique, homocyclique ou hétérocyclique,
**caractérisé en ce que**

a) dans la première phase de réaction, un auxiliaire chiral de la formule (VI)

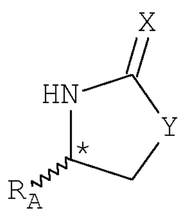
VI,

dans laquelle
X signifiant NH, NCN, 0 ou S,
Y signifiant NH, N($R^1$), 0 ou S, $R^1$ étant H, de l'alkyle ou de l'aryle,
$R_A$ signifiant de l'alkyle ou de l'aryle et
* étant noté pour une configuration R ou une configuration S,

est lié de façon covalente à l'atome de phosphore de chlorure de phosphoryle ou de chlorure de thiophosphoryle,
b) dans l'étape suivante, un alcool, un thiol, une amine est amené(e) en réaction en tant que nucléophile (Nu1), en présence d'une base avec le produit de la première phase de réaction et
c) dans la dernière étape, l'auxiliaire chiral (VI) est substitué par un nucléophile (Nu2).

**2.** Procédé selon la revendication 1, **caractérisé en ce que** dans l'étape suivante b), un stéréoisomère formé dans le surnageant est purifié et utilisé dans la dernière étape c).

**3.** Procédé selon la revendication 1 ou 2, **caractérisé en ce que** le nucléophile (Nu2) utilisé dans l'étape C est choisi dans le groupe composé de nucléoside, de monophosphate de nucléoside, de diphosphate de nucléoside, de triphosphate de nucléoside, d'analogue de nucléoside, d'analogue de monophosphate de nucléoside, d'analogue de diphosphate de nucléoside, d'analogue de triphosphate de nucléoside, de phosphate, de pyrophosphate, de phosphate de glycosyle et de glycosyle $\alpha$ déprotoné ou de sels de ces derniers.

**4.** Procédé selon la revendication 3, **caractérisé en ce qu'**on utilise de la 4-iso-propyl-2-mercapto-2-thiazoline en tant qu'auxiliaire chiral (VI) et **en ce que** sous formation de ($S_C$)-N-dichlorophosphoryl-4-isopropyl-2-mercapto-2-thiazoline, on la lie de façon covalente à l'atome de phosphore de chlorure de phosphoryle, **en ce qu'**on amène en réaction la($S_C$)-N-dichlorophosphoryl-4-isopropyl-2-mercapto-2-thiazoline dans l'étape suivante b) avec de l'alcool salicylique, **en ce qu'**on sépare les diastéréomères ($R_p$, $S_c$) et ($S_p$, $S_c$) obtenus et **en ce que** par la suite, l'auxiliaire chiral (VI) est substitué par un nucléoside sous formation de monophosphate de nucléoside cyclosaligényle issu du diastéréomère ($R_p$, $S_c$).

**5.** Procédé selon la revendication 3 ou 4, **caractérisé en ce que** le nucléoside est choisi dans le groupe comprenant l'adénosine, la guanosine, la cytidine, la thymidine, l'uridine, la désoxyadénosine, la désoxyguanosine, l'inosine, la désoxycytidine, la désoxyuridine, la désoxythymidine, la 2-thiocytidine, la $N^4$-acétyl-cytidine, la 2'-O-méthyl-cytidine, la 3-méthyl-cytidine, la 5-méthyl-cytidine, la 2-thiouridine, la pseudouridine, la dihydrouridine, la 5-(carboxyhydroxyméthyl)-uridine, la 5-carboxyméthylaminométhyl-uridine, la 5-méthylaminométhyl-uridine, la 5-méthoxy-carbonylméthyl-uridine, 5-méthoxy-uridine, la ribothymidine, la 1-méthyladénosine, la 2-méthyladénosine, la $N^6$-méthyladénosine, l'inosine, la 1-méthylinosine, la guanosine, la $N^2$-2,2-diméthyl-guanosine, la $N^2$-2-méthyl-guanosine, la $7^+$-méthyl-guanosine et la 2'-O-méthyl-guanosine.

**6.** Procédé selon l'une quelconque des revendications précédentes, **caractérisé en ce qu'**après l'étape suivante b), le pourcentage de surnageant de stéréomères est $\geq$ 10%, de préférence $\geq$ 20%, de manière préférée par ailleurs $\geq$ 30%, $\geq$ 40%, $\geq$ 50% ou $\geq$ 60% et de manière particulièrement préférée, $\geq$ 70%, $\geq$ 75%, $\geq$ 80%, $\geq$ 85% ou $\geq$ 90%.

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

### In der Beschreibung aufgeführte Nicht-Patentliteratur


- **C. MEIER.** *MiniRev. Med. Chem.,* 2002, vol. 2, 219-234
- **C. MEIER.** *Eur. J. Drg. Chem.,* 2006, 1081-1102
- **C. MEIER ; M. LOREY ; E. DE CLERCQ ; J. BALZARINI.** *J. Med. Chem.,* 1998, vol. 41, 14171427
- **S. C.R. HALL.** Phosphorus, Sulfur & related Elements. T.D. Inc, 1979, vol. 7, 171-84
- **S. RYU ; J.A. JACKSON ; C.M. THOMPSON.** *J. Org. Chem.,* 1991, vol. 56, 4999-5002
- **F. WU ; W.-S. LI ; M. CHEN-GOODSPEED ; M.A. SOGORB ; F.M. RAUSHEL.** *J. Am. Chem. Soc.,* 2000, vol. 122, 10206-07